(19) Europäisches Patentamt
European Patent Office
Office européen des brevets

(11) EP 2 806 949 B1

(12) EUROPÄISCHE PATENTSCHRIFT

(45) Veröffentlichungstag und Bekanntmachung des Hinweises auf die Patenterteilung:
**06.02.2019 Patentblatt 2019/06**

(21) Anmeldenummer: **12798313.8**

(22) Anmeldetag: **11.12.2012**

(51) Int Cl.:
***A61Q 5/06*** (2006.01) ***A61K 8/81*** (2006.01)
***A61K 8/73*** (2006.01)

(86) Internationale Anmeldenummer:
**PCT/EP2012/075010**

(87) Internationale Veröffentlichungsnummer:
**WO 2013/092285 (27.06.2013 Gazette 2013/26)**

# (54) MITTEL ZUR TEMPORÄREN VERFORMUNG KERATINISCHER FASERN AUF GRUNDLAGE EINER KOMBINATION SPEZIFISCHER FILMBILDENDER POLYMERE

AGENT FOR TEMPORARILY DEFORMING KERATIN FIBERS BASED ON A COMBINATION OF SPECIFIC FILM-FORMING POLYMERS

PRODUIT DE MISE EN FORME TEMPORAIRE DE FIBRES KÉRATINIQUES, À BASE D'UNE COMBINAISON DE POLYMÈRES FILMOGÈNES SPÉCIFIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.12.2011 DE 102011089172**

(43) Veröffentlichungstag der Anmeldung:
**03.12.2014 Patentblatt 2014/49**

(73) Patentinhaber: **Henkel AG & Co. KGaA**
**40589 Düsseldorf (DE)**

(72) Erfinder:
- **METTEN, Diane**
  **22393 Hamburg (DE)**
- **RICHTERS, Bernd**
  **21129 Hamburg (DE)**
- **SCHEFFLER, Rene**
  **25470 Ellerau (DE)**

(56) Entgegenhaltungen:
**EP-A1- 1 348 420**

- **DATABASE GNPD [Online] MINTEL; 1. April 2011 (2011-04-01), Redken: "Full Frame 07 Mousse", XP002728636, Database accession no. 1517864**
- **DATABASE GNPD [Online] MINTEL; 1. Mai 2008 (2008-05-01), L'Oréal: "Amped Up Mega Mousse", XP002728637, Database accession no. 905663**
- **DATABASE GNPD [Online] MINTEL; 1. September 2010 (2010-09-01), Matrix Essentials: "Firmfix Gel", XP002728638, Database accession no. 1384441**
- **Nn: "Hair Styling Cream with Natural Oils & Conditioners", , 30. November 2010 (2010-11-30), Seiten 1-2, XP055135296, Gefunden im Internet: URL:http://www.lubrizol.com/PersonalCare/S-X0007-Hair-Styling-Cream-with-Natural-Oils-Conditioners.pdf [gefunden am 2014-08-19]**
- **"CELQUAT L-200 (Polyquaternium-4)", , 1. Februar 2004 (2004-02-01), Seiten 1-21, XP055135400, Gefunden im Internet: URL:http://www.ptdju.com/resources/external/65D3886E-4434-4D8A-9088-CFE2C29E4C7F/Per sonal Care/Skin Care Application/Conditioning Agent/Brochure/Celquat L-200 document bundle.pdf [gefunden am 2014-08-19]**
- **Nn: "Fixate (TM) Polymer Comparison Chart", , 9 October 2007 (2007-10-09), pages 1-1, XP055312204, Retrieved from the Internet: URL:https://lubrizol.com/Personal-Care/Documents/Technical-Data-Sheets/TDS-624-Fixate(TM)-Polymer-Comparison-Chart.pdf [retrieved on 2016-10-19]**
- **HANS LAUTENSCHLÄGER: "INCI - Declaration", KOSMETIK INTERNATIONAL,, 1 January 2002 (2002-01-01), pages 50-53, XP007921002,**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Bemerkungen:

Die Akte enthält technische Angaben, die nach dem Eingang der Anmeldung eingereicht wurden und die nicht in dieser Patentschrift enthalten sind.

## Beschreibung

Die vorliegende Anmeldung beschreibt kosmetische Mittel auf Grundlage einer spezifischen Polymerkombination, die Verwendung dieser kosmetischen Mittel zur temporären Verformung keratinischer Fasern sowie kosmetische Verfahren unter Einsatz dieser Mittel.

Der Einsatz von Polymeren in verschiedensten kosmetischen Mitteln ist weit verbreitet. Sie finden sich in Mitteln zur Behandlung der Haut ebenso wie in Mitteln zur Behandlung der Haare, in Mitteln, die unmittelbar nach der Anwendung wieder ab- bzw. ausgewaschen werden, so genannten Rinse-off Produkten, genauso wie in Mitteln, die auf Haut oder Haar verbleiben, so genannten Leave-on Mitteln. Dabei werden die Polymere aus unterschiedlichsten Gründen eingesetzt und jeweils bestimmte Eigenschaften der Polymere ausgenutzt. In Mitteln zur Hautbehandlung, in Shampoos, Haarspülungen und Haarkuren stehen oftmals die verdickenden oder pflegenden Eigenschaften der Polymere im Vordergrund. In Mitteln zur temporären Verformung keratinischer Fasern, im Folgenden auch Stylingmittel genannt, sind neben diesen Eigenschaften vor allem filmbildende und/oder festigende Wirkungen gefragt. Oftmals dienen Polymere auch als Hilfsmittel um die Abscheidung und Fixierung anderer Wirk- und Inhaltsstoffe auf der Haut oder dem Haar zu verbessern oder erst möglich zu machen. So lassen sich beispielsweise durch Zusatz geeigneter Polymere zu Haarfärbemitteln die Reibechtheiten und die Beständigkeit der Färbung erhöhen.

In der Regel enthalten kosmetische Mittel einzelne Polymere, die speziell darauf zugeschnitten sind, einen ganz bestimmten Effekt zu erzielen. Sollen verschiedene Effekte erzielt werden, ist die Zugabe mehrerer Polymere erforderlich. Werden allerdings zu viele verschiedene Polymere eingesetzt, kann das eine Reihe von Nachteilen mit sich bringen. So können Probleme bei der Formulierung entstehen, etwa weil die Polymere untereinander oder mit anderen Bestandteilen des Mittels reagieren und es zu Ausfällungen oder Zersetzungen kommt. Bestimmte Polymere neigen auch dazu, sich auf der Haut und insbesondere auf dem Haar so beständig abzuscheiden, dass sie bei einer gewöhnlichen Wäsche nicht mehr vollständig entfernt werden und es zu einer unerwünschten Anreicherung des Polymers und damit letztlich einer Belastung von Haut oder Haar kommt.

Es besteht daher ständig Bedarf an Polymeren oder geeigneten Kombinationen weniger Polymere, die möglichst viele der gewünschten Eigenschaften gleichzeitig aufweisen.

Beispielsweise ist es im Falle der Stylingmittel notwendig, dass die eingesetzten Polymeren dem behandelten Haar einen möglichst starken Halt geben. Neben einem hohen Haltegrad müssen Stylingmittel jedoch eine ganze Reihe weiterer Anforderungen erfüllen. Diese können grob in Eigenschaften am Haar, Eigenschaften der jeweiligen Formulierung, z.B. Eigenschaften des Schaums, des Gels oder des versprühten Aerosols, und Eigenschaften, die die Handhabung des Stylingmittels betreffen, unterteilt werden, wobei den Eigenschaften am Haar besondere Wichtigkeit zukommt. Zu nennen sind insbesondere Feuchtebeständigkeit, niedrige Klebrigkeit und ein ausgewogener Konditioniereffekt. Weiterhin soll ein Stylingmittel möglichst für alle Haartypen universell einsetzbar sein. Handelt es sich bei dem Stylingmittel um ein Gel oder eine Paste, sollen die Polymere zudem verdickende Eigenschaften besitzen.

Aus der EP 1348420 A1 sind kosmetische Zusammensetzungen bekannt, die ein amphiphiles Polymer, ein kationisches Polymer und ein anionisches Tensid umfassen. Das amphiphile Polymer weist Blocksequenzen auf und ist verzweigt.

Die Haarstylingprodukte "Full Frame 07 Mousse" (Redken) und "Amped Up Mega Mousse" (L'Oreal) enthalten jeweils ein Polymer mit der INCI-Bezeichnung "AMP-acrylates/allyl Methacrylate Copolymer" sowie Polyquaternium-4.

Das Haarstylingprodukt "Firmfix Gel" (Matrix Essentials) enthält ein Polymer mit der INCI-Bezeichnung "AMP-acrylates/allyl Methacrylate Copolymer".

Die Aufgabe der vorliegenden Erfindung war es daher, weitere geeignete Polymerkombinationen zur Verfügung zu stellen, welche sich durch gute filmbildende und/oder festigende Eigenschaften auszeichnen, einen sehr hohen Haltegrad besitzen ohne dass dabei auf Flexibilität und gute Feuchtebeständigkeit - insbesondere Schweiß- und Wasserbeständigkeit - verzichtet werden müsste und sich zudem für die Herstellung stabil viskoser sowie stabil transparenter kosmetischer Zusammensetzungen eignen.

Diese Aufgaben wurden durch eine spezielle Polymerkombination gelöst. Ein erster Gegenstand der Erfindung ist daher ein kosmetisches Mittel zur temporären Verformung keratinischer Fasern, enthaltend in einem kosmetisch akzeptablen Träger

a) mindestens ein Copolymer A des Allylmethacrylats mit

- einem oder mehreren Monomeren a1) ausgewählt aus Acrylsäure und Methacrylsäure; und
- einem oder mehreren Monomeren a2) ausgewählt aus Acrylsäureester und Methacrylsäureester,

wobei der Gewichtsanteil des Copolymers A am Gesamtgewicht des Mittels 0,2 bis 5,0 Gew.-% beträgt;

b) mindestens eine polymere quartäre Ammoniumverbindung B aus der Gruppe der quarternierten Cellulosen, wobei das Copolymer B ausgewählt ist aus der Gruppe der Propfcopolymere der Hydroxyethylcellulose mit Diallyldimethylammoniumchlorid und wobei der Gewichtsanteil des Copolymers B am Gesamtgewicht des Mittels 0,2 bis 5,0 Gew.-% beträgt,

wobei es weiterhin mindestens eine monomere quartäre Ammoniumverbindung aus der Gruppe der Trimethylalkylammoniumhalogenide in einem Gewichtsanteil am Gesamtgewicht des Mittels von 0,2 bis 1,0 Gew.-% enthält.
Die erfindungsgemäßen Mittel enthalten die Wirkstoffe in einem kosmetischen Träger. Dieser kosmetische Träger ist im Sinne der Erfindung ist wässrig, alkoholisch oder wässrig-alkoholisch. Unter wässrig-alkoholischen Trägern sind im Sinne der vorliegenden Erfindung wasserhaltige Zusammensetzungen, enthaltend 3 bis 70 Gew.-% eines $C_1$-$C_4$-Alkohols, bezogen auf das Gesamtgewicht der Anwendungsmischung, insbesondere Ethanol bzw. Isopropanol, zu verstehen. Ein wässriger Träger enthält im Sinne der Erfindung mindestens 30 Gew.-%, insbesondere mindestens 50 Gew.-% Wasser, bezogen auf das Gesamtgewicht der Anwendungsmischung. Bevorzugte kosmetische Mittel enthalten, bezogen auf sein Gesamtgewicht, 40 bis 99 Gew.-%, vorzugsweise 50 bis 98 Gew.-%, besonders bevorzugt 60 bis 95 Gew.-% und insbesondere 70 bis 90 Gew.-% Wasser. Der pH-Wert (10%-ige Lösung, 20°C) bevorzugter kosmetischer Mittel beträgt 4 bis 9, vorzugsweise 5 bis 8 und insbesondere von 6 bis 7.

Die erfindungsgemäßen Mittel enthaltend als ersten wesentlichen Bestandteil ein Copolymer A des Allylmethacrylats mit

- einem oder mehreren Monomeren a1) ausgewählt aus Acrylsäure und Methacrylsäure; und
- einem oder mehreren Monomeren a2) ausgewählt aus Acrylsäureester und Methacrylsäureester.

Vorzugsweise handelt es sich bei den genannten Acrylsäureestern und Methacrylsäureestern um $C_1$-$C_{12}$-Alkylacrylate und $C_1$-$C_{12}$-Alkylmethacrylate, besonders bevorzugt um Methylacrylat, Ethylacrylat, Propylacrylat, Methylmethacrylat, Ethylmethacrylat, Propylmethacrylat und deren Mischungen.

Für die technische Wirkung der erfindungsgemäßen Mittel hat es sich als vorteilhaft erwiesen, wenn das Copolymer A zu mindestens 70 Gew.-%, vorzugsweise zu mindestens 80 Gew.-%, bevorzugt zu mindestens 90 Gew.-% und insbesondere zu mindestens 95 Gew.-% auf Allylmethacrylat und einem oder mehreren Monomeren aus der Gruppe Acrylsäure, Methacryläsure, Acrylsäureester und Methacrylsäureester basiert.

Technich besonders bevorzugt ist es, wenn das Copolymer A zu mindestens 70 Gew.-%, vorzugsweise zu mindestens 80 Gew.-%, bevorzugt zu mindestens 90 Gew.-% und insbesondere zu mindestens 95 Gew.-% auf

- Allylmethacrylat und
- einem oder mehreren Monomeren aus der Gruppe Acrylsäure, Methacryläsure und
- einem oder mehreren Monomeren aus der Gruppe Acrylsäureester und Methacrylsäureester

basiert.

Ein beispielhaftes Copolymer A stellt das Aminomethylpropanolsalz von Copolymeren des Allylmethacrylats mit einem oder mehreren Monomeren ausgewählt aus Acrylsäure, Methacrylsäure, Acrylsäureester und Methacrylsäureester dar. Ein entsprechendes Copolymer mit der INCI-Bezeichnung AMP-Acrylates/Allyl Methacrylate Copolymer wird von der Firma Noveon unter der Bezeichnung Fixate™ G-100 vertrieben.

Der Gewichtsanteil des Copolymers A am Gesamtgewicht erfindungsgemäßer kosmetischer Mittel beträgt 0,2 bis 5,0 Gew.-%.
Die erfindungsgemäßen Mittel enthaltend als zweiten wesentlichen Bestandteil eine polymere quartäre Ammoniumverbindung B aus der Gruppe der quarternierten Cellulosen. Die quarternierten Cellulosen werden durch "Propfung" erhalten. Es wird Hydroxyethylcellulose, die mit einem Diallyldimethylammoniumsalz gepfropft ist, eingesetzt. Handelsprodukte, die dieser Definition entsprechen, sind insbesondere die Produkte mit der Bezeichnung Celquat® L 200 (INCI Name: Polyquaternium-4) und Celquat® H 100 (INCI Name: Polyquaternium-4) von der Firma National Starch.
In Bezug auf die kosmetischen Eigenschaften dieser Mittel haben sich die unter der INCI Bezeichnung Polyquaternium-4 zusammengefassten quaternierten Cellulosen als besonders vorteilaft erwiesen.
Besonders bevorzugt sind insbesondere solche Copolymere B, die zu mindestens 70 Gew.-%, vorzugsweise zu mindestens 80 Gew.-%, bevorzugt zu mindestens 90 Gew.-% und insbesondere zu mindestens 95 Gew.-% auf Hydroxyethylcellulose und Diallyldimethylammoniumchlorid basieren.

Der Gewichtsanteil der polymeren quartären Ammoniumverbindung B aus der Gruppe der quarternierten Cellulosen am Gesamtgewicht erfindungsgemäßer kosmetischer Mittel beträgt 0,2 bis 5,0 Gew.-%.
Die erfindungsgemäßen Mittel zeichnen sich von kosmetischen Mitteln mit alternativen polymeren quartären Ammoniumverbindungen neben den oben genannten Vorteilen insbesondere auch durch einen verbesserten Haltegrad aus. Als für die kosmetischen Eigenschaften der erfindungsgemäßen Mittel besonders vorteilhaft hat sich ein Gewichtsverhältnis der Polymere A und B in dem kosmetischen Mittel zwischen 8:1 und 1:8, vorzugsweise zwischen 6:1 und 1:6 und insbesondere zwischen 4:1 und 1:4 erwiesen.

Das Copolymer A wird in dem kosmetischen Mitteln vorzugsweise in teilneutraliserter oder neutralisierter Form eingesetzt. Zur Neutralisation wird bevorzugt mindestens ein Alkanolamin verwendet. Die als erfindungsgemäßes Alkalisierungsmittel einsetzbaren Alkanolamine werden bevorzugt ausgewählt aus primären Aminen mit einem $C_2$-$C_6$-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), Tris(2-hydroxyethyl)-amin (Triethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine werden ausgewählt aus der Gruppe 2-Aminoethan-1-ol, 2-Amino-2-methylpropan-1-ol und 2-Amino-2-methyl-propan-1,3-diol. Als besonders geeignetes Neutralisationsmittel hat sich dabei 2-Amino-2-methylpropanol erwiesen. Erfindungsgemäß bevorzugte kosmetische Mittel enthalten mindestens ein Alkanolamin, vorzugsweise 2-Amino-2-methylpropanol. Das 2-Amino-2-methylpropanol wird in den erfindungsgemäßen Mitteln vorzugsweise in einer Menge eingesetzt, welche die zur Neutralisation des Copolymers A benötigte Menge nicht überschreitet. Vorzugsweise beträgt die in den erfindungsgemäßen Mitteln eingesetzte Mengen an 2-Amino-2-methylpropanol 80 bis 100%, besonders bevorzugt 90 bis 100% und insbesondere 95 bis 100% der zur vollständigen Neutralisation des Copolymers A benötigten Menge. In einer bevorzugten Ausführungsform beträgt der Gewichtsanteil des 2-Amino-2-methylpropanols am Gesamtgewicht des kosmetischen Mittels 0,1 bis 4,0 Gew.-%, bevorzugt 0,2 bis 3,0 Gew.% und insbesondere 0,5 bis 2,0 Gew.-%.

Neben den zuvor beschriebenen Copolymeren und Trägersubstanzen können die erfindungsgemäßen kosmetischen Mittel weitere Inhaltsstoffe enthalten. Zur Gruppe dieser weiteren Inhaltsstoffe zählen insbesondere die kosmetisch wirksamen Hilfs- und Zusatzstoffe.

Die erfindungsgemäßen kosmetischen Mittel enthalten mindestens eine quartäre Ammoniumverbindung aus der Gruppe der Trimethylalkylammoniumhalogenide in einem Gewichtsanteil am Gesamtgewicht des Mittels von 0,2 bis 1,0 Gew.-%. Zur Gruppe der Trimethylalkylammoniumhalogenide zählen insbesondere die Verbindungen der Formel (Tkat1-1).

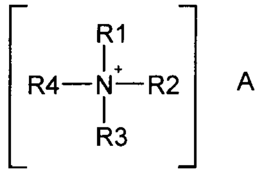

(Tkat1)

In der Formel (Tkat1) stehen R1, R2, R3 und R4 für jeweils unabhängig voneinander für Wasserstoff, eine Methylgruppe, eine Phenylgruppe, eine Benzylgruppe, für einen gesättigten, verzweigten oder unverzweigten Alkylrest mit einer Kettenlänge von 8 bis 30 Kohlenstoffatomen, welcher gegebenenfalls mit einer oder mehreren Hydroxygruppen substituiert sein kann. A steht für ein physiologisch verträgliches Anion, beispielsweise Halogenide wie Chlorid oder Bromid sowie Methosulfate.

Beispiele für Verbindungen der Formel (Tkat1) sind Lauryltrimehtylammoniumchlorid, Cetyltrimethylammoniumchlorid, Cetyltrimethylammoniumbromid, Cetyltrimethylammoniummethosulfat, Dicetyldimethylammoniumchlorid, Tricetylmethylammoniumchlorid, Stearyltrimethylammoniumchlorid, Distearyldimethylammoniumchlorid, Lauryldimethylbenzylammoniumchlorid, Behenyltrimethylammoniumchlorid, Behenyltrimethylammoniumbromid, Behenyltrimethylammoniummethosulfat.

Als geeignete Hilfs- und Zusatzstoffe sind insbesondere zusätzliche Pflegestoffe zu nennen.

Als Pflegestoff einer anderen Verbindungsklasse kann das Mittel beispielsweise mindestens ein Proteinhydrolysat und/oder eines seiner Derivate enthalten. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Als Pflegestoff kann das erfindungsgemäße Mittel weiterhin mindestens ein Vitamin, ein Provitamin, eine Vitaminvorstufe und/oder eines derer Derivate enthalten. Dabei sind erfindungsgemäß solche Vitamine, Provitamine und Vitaminvorstufen bevorzugt, die üblicherweise den Gruppen A, B, C, E, F und H zugeordnet werden.

Wie auch der Zusatz von Glycerin und/oder Propylenglykol erhöht der Zusatz von Panthenol die Flexibilität des bei Anwendung des erfindungsgemäßen Mittels gebildeten Polymerfilms.

Als Pflegestoff können die erfindungsgemäßen Mittel weiterhin mindestens einen Pflanzenextrakt, aber auch Mono- bzw. Oligosaccharide und/oder Lipide enthalten.

Weiterhin sind als Pflegestoff Ölkörper geeignet. Zu den natürlichen und synthetischen kosmetischen Ölkörpern

sind beispielsweise zu zählen pflanzliche Öle, flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen. Bevorzugte erfindungsgemäße kosmetische Mittel enthalten mindestens einen Ölkörper, vorzugsweise mindestens einen Ölkörper aus der Gruppe der Silikonöle. Zur Gruppe der Silikonöle zählen insbesondere die Dimethicone, zu welchen auch die Cyclomethicone zu rechnen sind, die aminofunktionellen Silikone sowie die Dimethiconole. Die Dimethicone können sowohl linear als auch verzweigt als auch cyclisch oder cyclisch und verzweigt sein. Geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane.

Esteröle, das heißt Ester von $C_6$ - $C_{30}$ - Fettsäuren mit $C_2$ - $C_{30}$ - Fettalkoholen, vorzugsweise Monoester der Fettsäuren mit Alkoholen mit 2 bis 24 C-Atomen wie beispielsweise Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkohol-caprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V) sind weitere bevorzugte pflegende Ölkörper.

Als Pflegestoffe eignen sich weiterhin Dicarbonsäureester, symmetrische, unsymmetrische oder cyclische Ester der Kohlensäure mit Fettalkoholen, Trifettsäureester von gesättigten und/oder ungesättigten linearen und/oder verzweigten Fettsäuren mit Glycerin oder Fettsäurepartialglyceride, worunter Monoglyceride, Diglyceride und deren technische Gemische zu verstehen sind.

In Bezug auf die kosmetische Wirkung haben sich kosmetische Mittel als vorteilhaft erwiesen, bei denen der Gewichtsanteil des Ölkörpers am Gesamtgewicht des Mittels 0,01 bis 5,0 Gew.-%, vorzugsweise 0,02 bis 4,0 Gew.-% und insbesondere 0,05 bis 2,0 Gew.-% beträgt.

Die Zusammensetzung einiger bevorzugter kosmetischer Mittel kann den folgenden Tabellen entnommen werden (Angaben in Gew.-% bezogen auf das Gesamtgewicht des kosmetischen Mittels sofern nicht anders angegeben).

| | Formel 1 | Formel 2 | Formel 3 | Formel 4 | Formel 5 |
|---|---|---|---|---|---|
| Copolymer A 1) | 0,05 bis 10 | 0,1 bis 7,0 | 0,2 bis 5,0 | 0,4 | 1,2 |
| Copolymer B 2) | 0,05 bis 10 | 0,1 bis 7,0 | 0,2 bis 5,0 | 0,8 | 0,8 |
| Alkyltrimethylammoniumchlorid | 0,05 bis 3,0 | 0,1 bis 2,0 | 0,2 bis 1,0 | 0,5 | 0,3 |
| Wasser, Misc | add 100 | add 100 | add 100 | add 100 | add 100 |
| | Formel 6 | Formel 7 | Formel 8 | Formel 9 | Formel 10 |
| Copolymer A 1) | 0,05 bis 10 | 0,1 bis 7,0 | 0,2 bis 5,0 | 0,4 | 1,2 |
| Copolymer B 2) | 0,05 bis 10 | 0,1 bis 7,0 | 0,2 bis 5,0 | 0,8 | 0,8 |
| Alkyltrimethylammoniumchlorid | 0,05 bis 3,0 | 0,1 bis 2,0 | 0,2 bis 1,0 | 0,5 | 0,3 |
| Wasser | 40 bis 99 | 50 bis 98 | 60 bis 95 | 97 | 92 |
| Misc | add 100 | add 100 | add 100 | add 100 | add 100 |

1) Copolymer A, welches zu mindestens 95 Gew.-% auf

- Allylmethacrylat und
- einem oder mehreren Monomeren aus der Gruppe Acrylsäure, Methacryläsure und
- einem oder mehreren Monomeren aus der Gruppe Acrylsäureester und Methacrylsäureester basiert.

2) Propfcopolymer der Hydroxyethylcellulose mit Diallyldimethylammoniumchlorid

Die Formulierung der erfindungsgemäßen Mittel kann in allen für kosmetische Mittel üblichen Formen erfolgen, beispielsweise in Form von Lösungen, die beispielsweise als Haarwasser auf das Haar aufgebracht werden können, in Form von Cremes, Emulsionen, Wachsen, Gelen oder auch tensidhaltigen schäumenden Lösungen oder anderen Zubereitungen, die für die Anwendung auf dem Haar geeignet sind. In einer alternativen Ausführungsform können diese Mittel jedoch auch in Gel- oder in Cremeform vorliegen, wobei transparente Gele besonders bevorzugt sind.
Die erfindungsgemäßen Zusammensetzungen eignen sich insbesondere sehr gut dazu, Gasblasen im Mittel zu stabilisieren. Auf diese Weise können Luft oder andere Gase bzw. Gasgemische gut und langzeitstabil in die erfindungsgemäßen Mittel eingearbeitet werden. Dies kann wahlweise bei der Herstellung der Mittel geschehen, indem das Mittel vor der Abfüllung mit Gas, vorzugsweise Luft, beaufschlagt wird und ein Produkt abgefüllt wird, das sichtbare Gasblasen enthält. Bevorzugt liegen die erfindungsgemäßen Mittel in Form eines Schaums vor. Als Schaum wird dabei eine Zubereitung bezeichnet, die Gas gefüllte Bläschen aufweist, welche von flüssigen (flüssiger Schaum) oder festen (fester Schaum) Wänden umgeben sind. Bei den in der nachfolgenden Tabelle angeführten Zusammensetzungen handelt es sich vorzugsweise um feste Schäume. Die Dichte bevorzugter Zusammensetzungen beträgt 0,3 bis 1,0 g/$cm^3$, vorzugsweise 0,4 bis 0,9 g/$cm^3$ und insbesondere 0,5 bis 0,8 g/$cm^3$. Die Herstellung solcher Schäume kann beispielsweise durch Aufschlagen der Zubereitung in einem geeigneten Mischer oder durch Beaufschlagung mit einem geeigneten Gas, vorzugsweise Luft, erfolgen.
Die Zusammensetzung einiger bevorzugter kosmetischer Schäume kann der folgenden Tabellen entnommen werden. In dieser Tabelle verweist die linke Spalte ("Formel x") auf jeweils eine der in den weiter oben offenbarten Tabellen angeführten beispielhaften kosmetischen Zusammensetzungen. Die weiteren Spalten zwei bis sieben ("Dichte") geben jeweils die Dichte der entsprechenden kosmetischen Zusammensetzung an.

Eine kosmetische Zubereitung gemäß Zeile 2, Spalte 5 der nachfolgenden Tabelle, umfasst mit anderen Worten ein kosmetisches Mittel gemäß Formel 1 mit einer Dichte von 0,44 g/$cm^3$.

| | Dichte [g/$cm^3$] | | | | | |
|---|---|---|---|---|---|---|
| Formel 1 | 0,3 bis 1,0 | 0,93 | 0,4 bis 0,9 | 0,44 | 0,5 bis 0,8 | 0,61 |
| Formel 2 | 0,3 bis 1,0 | 0,93 | 0,4 bis 0,9 | 0,44 | 0,5 bis 0,8 | 0,61 |
| Formel 3 | 0,3 bis 1,0 | 0,93 | 0,4 bis 0,9 | 0,44 | 0,5 bis 0,8 | 0,61 |
| Formel 4 | 0,3 bis 1,0 | 0,93 | 0,4 bis 0,9 | 0,44 | 0,5 bis 0,8 | 0,61 |
| Formel 5 | 0,3 bis 1,0 | 0,93 | 0,4 bis 0,9 | 0,44 | 0,5 bis 0,8 | 0,61 |
| Formel 6 | 0,3 bis 1,0 | 0,93 | 0,4 bis 0,9 | 0,44 | 0,5 bis 0,8 | 0,61 |
| Formel 7 | 0,3 bis 1,0 | 0,93 | 0,4 bis 0,9 | 0,44 | 0,5 bis 0,8 | 0,61 |
| Formel 8 | 0,3 bis 1,0 | 0,93 | 0,4 bis 0,9 | 0,44 | 0,5 bis 0,8 | 0,61 |
| Formel 9 | 0,3 bis 1,0 | 0,93 | 0,4 bis 0,9 | 0,44 | 0,5 bis 0,8 | 0,61 |
| Formel 10 | 0,3 bis 1,0 | 0,93 | 0,4 bis 0,9 | 0,44 | 0,5 bis 0,8 | 0,61 |

In einer alternativen Ausführungsform können die erfindungsgemäßen kosmetischen Mittel als Pump- oder Aerosolspray konfektioniert werden. Bevorzugte Aerosolsprays enthalten dabei neben weiteren Aktiv- und Hilfsstoffen ein Treibmittel. Erfindungsgemäß geeignete Treibmittel (Treibgase) sind Propan, n-Butan, iso-Butan, Dimethylether (DME), Stickstoff, Luft, Lachgas, 1,1-Difluorethan, und zwar sowohl einzeln als auch in Kombination. Auch hydrophile Treibgase, wie z. B. Kohlendioxid, können vorteilhaft im Sinne der vorliegenden Erfindung eingesetzt werden, wenn der Anteil an hydrophilen Gasen gering gewählt wird und lipophiles Treibgas (z. B. Propan/Butan) im Überschuss vorliegt. Besonders bevorzugt sind Dimethylether, Propan, n-Butan, iso-Butan sowie Mischungen dieser Treibgase. Ganz besonders bevorzugt ist der Einsatz von Propan/Butan Gemischen oder Isobutan. Kosmetische Mittel, die das Treibmittel bezogen auf ihr Gesamtgewicht in einer Menge von 2,0 - 20 Gew.%, bevorzugt 4,0 - 15 Gew.% und besonders bevorzugt 5,0 - 10 Gew.-% enthalten, sind erfindungsgemäß bevorzugt.

Die Zusammensetzung einiger bevorzugter Treibmittel-haltiger kosmetischer Mittel kann der folgenden Tabellen entnommen werden. In dieser Tabelle verweist die linke Spalte ("Formel x") auf jeweils eine der in den weiter oben offenbarten Tabellen angeführten beispielhaften kosmetischen Zusammensetzungen. Die weiteren Spalten zwei bis sieben ("Treibmittel") geben jeweils die der entsprechenden kosmetischen Zusammensetzung zugesetzte Menge an Treibmittel an. Diese Angaben in "Gew.-%" beziehen sich auf das Gesamtgewicht der kosmetischen Zusammensetzung der jeweiligen "Formel x" ohne Treibmittel.

Eine kosmetische Zubereitung gemäß Zeile 2, Spalte 5 der nachfolgenden Tabelle, umfasst mit anderen Worten eine 20:1 Mischung des Treibmittel-freien kosmetischen Mittels gemäß Formel 1 mit einem Propan/Butan Gemisch.

| | Treibmittel [Gew.-%] | | | | | |
|---|---|---|---|---|---|---|
| Formel 1 | 2,0 bis 10 | 5,0 | 2,0 bis 10 P/B* | 5,0 P/B | 2,0 bis 10 iB* | 5,0 iB |
| Formel 2 | 2,0 bis 10 | 5,0 | 2,0 bis 10 P/B* | 5,0 P/B | 2,0 bis 10 iB* | 5,0 iB |
| Formel 3 | 2,0 bis 10 | 5,0 | 2,0 bis 10 P/B* | 5,0 P/B | 2,0 bis 10 iB* | 5,0 iB |
| Formel 4 | 2,0 bis 10 | 5,0 | 2,0 bis 10 P/B* | 5,0 P/B | 2,0 bis 10 iB* | 5,0 iB |
| Formel 5 | 2,0 bis 10 | 5,0 | 2,0 bis 10 P/B* | 5,0 P/B | 2,0 bis 10 iB* | 5,0 iB |
| Formel 6 | 2,0 bis 10 | 5,0 | 2,0 bis 10 P/B* | 5,0 P/B | 2,0 bis 10 iB* | 5,0 iB |
| Formel 7 | 2,0 bis 10 | 5,0 | 2,0 bis 10 P/B* | 5,0 P/B | 2,0 bis 10 iB* | 5,0 iB |
| Formel 8 | 2,0 bis 10 | 5,0 | 2,0 bis 10 P/B* | 5,0 P/B | 2,0 bis 10 iB* | 5,0 iB |
| Formel 9 | 2,0 bis 10 | 5,0 | 2,0 bis 10 P/B* | 5,0 P/B | 2,0 bis 10 iB* | 5,0 iB |
| Formel 10 | 2,0 bis 10 | 5,0 | 2,0 bis 10 P/B* | 5,0 P/B | 2,0 bis 10 iB* | 5,0 iB |

* "P/B" entspricht einem Propan/Butan Gemisch
** "iB" entspricht Isobutan

Wie eingangs ausgeführt, weisen die erfindungsgemäßen Mittel vorteilhafte haarfixierende Eigenschaften auf. Ein Verfahren zur temporären Verformung keratinischer Fasern, bei welchem eine erfindungsgemäße Zusammensetzung auf die keratinische Faser aufgebracht wird, ist daher ein weiterer Gegenstand der vorliegenden Anmeldung. Die Verwendung eines erfindungsgemäßen kosmetischen Mittels zur temporären Verformung keratinischer Fasern ist ein weiterer Gegenstand der vorliegenden Anmeldung. Wie eingangs ausgeführt, zeichnen sich die erfindungsgemäßen Mittel insbesondere durch einen verbesserten Halt bei der temporären Verformung keratinischer Fasern aus. Ein zusätzlicher Gegenstand der vorliegenden Anmeldung ist daher die Verwendung eines erfindungsgemäßen kosmetischen Mittels zur Verbesserung des Halts bei der temporären Verformung keratinischer Fasern.

Beispiele

Es wurde die Feuchtebeständigkeit (High Humidity Curl Retention; HHCR) der mittels der nachfolgenden drei haarkosmetischen Mittel erzielten temporären Haarverformung bestimmt.

| | **E1** | **V1** | **V2** |
|---|---|---|---|
| Fixate G-100 [1] | 4,80 | 5,77 | -- |
| Celquat L-200 [2] | 0,75 | -- | 1,6 |
| Natriumbenzoat | 0,3 | 0,3 | 0,3 |
| D-Panthenol (75%) | 0,2 | 0,2 | 0,2 |
| Dow Corning 939 [3] | 0,2 | 0,2 | 0,2 |
| Dehyquart A CA [4] | 1,0 | 1,0 | 1,0 |
| Castor Oil hydrogenated, 40 EO | 0,2 | 0,2 | 0,2 |
| Parfüm | 0,1 | 0,1 | 0,1 |
| Wasser, Misc | add 100 | add 100 | add 100 |

[1] Copolymer mit der INCI-Bezeichnung AMP-Acrylates/Allyl Methacrylate Copolymer (26% in Wasser)
[2] modifizierte Hydroxyalkylcellulose (INCI: Polyquaternium-4, 94%)
[3] Silikon Zubereitung (INCI: Amodimethicone, Trideceth-12, Cetrimonium Chloride)
[4] Trimethylhexadecylammoniumchlorid

Zur Bestimmung der High Humidity Curl Retention wurden standardisierte Haarsträhnen der Fa. Kerling (Art. Nr. 827560) des Haartyps "European Natural, Farbe 6/0) von einer Länge ($L_{max}$) von 220 mm und einem Gewicht von 0.6 g eingesetzt. Zur Vorbereitung wurden die Strähnen mit einer 12.5 Gew.-%igen Natriumlaurethsulphat-Lösung gewaschen. Die Haarsträhnen wurden über Nacht in einem Trockenofen bei 318 K getrocknet.

0.18 g der Zusammensetzungen wurden auf jeweils eine Haarsträhne appliziert und einmassiert. Die Strähne wurde sodann auf einen Wickler gewickelt (Fripac-medis, Ø 7mm, Art. Nr. D-1203) und über Nacht bei Raumtemperatur getrocknet.

Die Wickler wurden vorsichtig entfernt und die Strähne aufgehängt. Die Längen der Locken wurden jeweils gemessen ($L_0$) und die Strähnen in eine Klimakammer gegeben. Dort wurden sie bei 294 K und einer relativen Luftfeuchtigkeit von 85% über einen Zeitraum von 6 h gelagert und danach die Längen der Locken erneut vermessen ($L_t$).

Pro Zusammensetzung wurden 5 Teststrähnen entsprechend behandelt und vermessen.

Die High-Humidity-Curlretention (HHCR) wurde nach folgender Formel berechnet und für jede Zusammensetzung das arithmetische Mittel aus den HHCR-Werten der 5 Teststrähnen gebildet:

$$HHCR = \frac{L_{\max} - L_t}{L_{\max} - L_0}$$

| | E1 | V1 | V2 |
|---|---|---|---|
| HHCR | 77% | 53% | 71% |

Den Messdaten ist die synergistische Wirkung der erfindungsgemäßen Polymerkombination in Zusammensetzung E1 zu entnehmen.

## Patentansprüche

1. Kosmetisches Mittel zur temporären Verformung keratinischer Fasern, enthaltend in einem kosmetisch akzeptablen Träger

   a) mindestens ein Copolymer A des Allylmethacrylats mit

      - einem oder mehreren Monomeren a1) ausgewählt aus Acrylsäure und Methacrylsäure; und
      - einem oder mehreren Monomeren a2) ausgewählt aus Acrylsäureester und Methacrylsäureester,

   wobei der Gewichtsanteil des Copolymers A am Gesamtgewicht des Mittels 0,2 bis 5,0 Gew.-% beträgt

   b) mindestens eine polymere quartäre Ammoniumverbindung B aus der Gruppe der quarternierten Cellulosen, wobei das Copolymer B ausgewählt ist aus der Gruppe der Propfcopolymere der Hydroxyethylcellulose mit Diallyldimethylammoniumchlorid und wobei der Gewichtsanteil des Copolymers B am Gesamtgewicht des Mittels 0,2 bis 5,0 Gew.-% beträgt,

   wobei es weiterhin mindestens eine monomere quartäre Ammoniumverbindung aus der Gruppe der Trimethylalkylammoniumhalogenide in einem Gewichtsanteil am Gesamtgewicht des Mittels von 0,2 bis 1,0 Gew.-% enthält.

2. Kosmetisches Mittel nach Anspruch 1, **dadurch gekennzeichnet, dass** das kosmetische Mittel weiterhin mindestens ein Alkanolamin, vorzugsweise 2-Amino-2-methylpropanol enthält.

3. Kosmetisches Mittel nach einem der vorherigen Ansprüche, **dadurch gekennzeichnet, dass** es weiterhin mindestens einen Ölkörper, vorzugsweise mindestens einen Ölkörper aus der Gruppe der Silikonöle, enthält.

4. Verwendung eines kosmetischen Mittels nach einem der vorherigen Ansprüche, zur temporären Verformung keratinischer Fasern.

5. Verfahren zur temporären Verformung keratinischer Fasern, bei welchem eine Zusammensetzung nach einem der Ansprüche 1 bis 3 auf die keratinische Faser aufgebracht wird.

## Claims

1. A cosmetic agent for temporarily shaping keratin fibers, containing, in a cosmetically acceptable carrier,

   a) at least one copolymer A of allyl methacrylate, comprising

   - one or more monomers a1) selected from acrylic acid and methacrylic acid; and
   - one or more monomers a2) selected from acrylic acid ester and methacrylic acid ester,

   wherein the weight proportion of copolymer A with respect to the total weight of the agent is 0.2 to 5.0 wt.%,
   b) at least one polymeric quaternary ammonium compound B
   from the group of
   quaternized celluloses, wherein copolymer B is selected from the group of graft copolymers of hydroxyethyl cellulose and diallyldimethylammonium chloride and wherein the weight proportion of copolymer B with respect to the total weight of the agent is 0.2 to 5.0 wt.%,

   wherein the agent further contains at least one monomeric quaternary ammonium compound from the group of trimethyl alkylammonium halides in a weight proportion with respect to the total weight of the agent of 0.2 to 1.0 wt.%.

2. The cosmetic agent according to claim 1, **characterized in that** the cosmetic agent also contains at least one alkanolamine, preferably 2-amino-2-methylpropanol.

3. The cosmetic agent according to one of the preceding claims, **characterized in that** it additionally contains at least one oil component, preferably at least one oil component from the group of silicone oils.

4. The use of a cosmetic agent according to one of the preceding claims for temporarily shaping keratin fibers.

5. A method for temporarily shaping keratin fibers in which a composition according to one of claims 1 to 3 is applied to the keratin fibers.

## Revendications

1. Agent cosmétique, destiné à pour la mise en forme temporaire de fibres kératiniques, contenant dans un support cosmétiquement acceptable

   a) au moins un copolymère A du méthacrylate d'allyle comportant

   - un ou plusieurs monomères a1) choisis parmi l'acide acrylique et l'acide méthacrylique ; et
   - un ou plusieurs monomères a2) choisis parmi un ester de l'acide acrylique et un ester de l'acide méthacrylique,

   la proportion en poids du copolymère A rapportée au poids total de l'agent étant de 0,2 à 5,0 % en poids
   b) au moins un composé d'ammonium quaternaire polymère B du groupe des celluloses quaternisées, le copolymère B étant choisi dans le groupe des copolymères greffés de l'hydroxyéthylcellulose avec du chlorure de diallyldiméthylammonium et la proportion en poids du copolymère B, rapportée au poids total de l'agent, étant de 0,2 à 5,0 % en poids,

   l'agent contenant en outre au moins un composé d'ammonium quaternaire monomère du groupe des halogénures de triméthylalkylammonium dans une proportion en poids, rapportée au poids total de l'agent, allant de 0,2 à 1,0 % en poids.

2. Agent cosmétique selon la revendication 1, **caractérisé en ce que** l'agent cosmétique contient en outre au moins une alcanolamine, de préférence du 2-amino-2-méthylpropanol.

3. Agent cosmétique selon une des revendications précédentes, **caractérisé en ce qu'**il contient en outre au moins une substance huileuse, de préférence au moins une substance huileuse du groupe des huiles de silicone.

**4.** Utilisation d'un agent cosmétique selon une des revendications précédentes pour la mise en forme temporaire de fibres kératiniques.

**5.** Procédé de mise en forme temporaire de fibres kératiniques dans lequel une composition selon une des revendications 1 à 3 est appliquée sur les fibres kératiniques.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1348420 A1